# EUROPÄISCHE PATENTSCHRIFT

(11) **EP 2 956 071 B1**
(45) Veröffentlichungstag und Bekanntmachung des Hinweises auf die Patenterteilung: **07.04.2021**
(21) Anmeldenummer: 13817682.1
(22) Anmeldetag: 20.12.2013
(51) Int. Cl.: A61B 17/34, A61N 1/05, A61M 25/06, A61M 19/00, A61M 25/00

(54) **SET FÜR DIE PERIPHERE NERVENBLOCKADE**
SET FOR PERIPHERAL NERVE BLOCKING
KIT POUR BLOCAGE NERVEUX PÉRIPHÉRIQUE

(30) Priorität: 15.02.2013 DE 102013101538
(43) Veröffentlichungstag der Anmeldung: 23.12.2015
(73) Patentinhaber: Pajunk GmbH Medizintechnologie, 78187 Geisingen (DE)
(72) Erfinder: PAJUNK, Horst, 78187 Geisingen (DE); PAJUNK, Heinrich, 78187 Geisingen (DE)
(74) Vertreter: Westphal, Mussgnug & Partner Patentanwälte mbB
(86) Internationale Anmeldenummer: PCT/EP2013/077618
(87) Internationale Veröffentlichungsnummer: WO 2014/124719

(56) Entgegenhaltungen:
- EP-A1- 0 564 859
- EP-A1- 1 002 500
- EP-A1- 1 849 493
- WO-A1-2008/082170
- US-A- 4 239 042
- US-A1- 2004 039 373
- US-A1- 2005 049 663
- US-A1- 2005 090 801
- US-A1- 2006 129 102
- US-A1- 2009 187 140

## Beschreibung

Die Erfindung betrifft ein Set für die periphere Nervenblockade gemäß dem Oberbegriff des Patentanspruchs 1.

Die periphere Nervenblockade wird für die operative oder analgetische Behandlung der Extremitäten verwendet. Dabei wird eine Kanüle in den Perineuralraum eingestochen und ein Katheter wird durch die Kanüle geführt in den Perineuralraum eingeschoben bis zu dem Bereich des Nervs, in welchem das Anästhetikum appliziert werden soll.

Aus der EP 1 002 500 A1 ist ein Set für die periphere Nervenblockade bekannt, welches eine starre Kanüle für den Einstich in den Perineuralraum aufweist. Die Positionierung der distalen Kanülenspitze kann dabei insbesondere durch elektrische Stimulation erfolgen. Auf die Kanüle ist eine äußere Katheterhülle aufschiebbar, die beim Einstich zusammen mit der Kanüle in den Körper des Patienten eingeführt wird. Sobald die Kanülenspitze den Zielort erreicht hat, wird die Kanüle herausgezogen und ein Innenkatheter wird durch die verbleibende Katheterhülle eingeschoben, bis sein distales Ende distal aus der Katheterhülle hinausragt. Durch den Innenkatheter kann dann ein Anästhetikum injiziert werden. Die Katheterhülle weist an ihrem proximalen Ende ein Luer-Lock-Konnektorteil auf zum Anschließen einer Spritze oder eines Schlauchkonnektors. Die axiale Position des Innenkatheters in der Katheterhülle wird durch Markierungen des Innenkatheters angezeigt. Um über den Innenkatheter eine Anästhetikum einleiten zu können, wird an dem proximalen Ende des Innenkatheters ein Anschlussadapter angesetzt.

Als weiterer Stand der Technik werden die US 2005/049663 A1, EP 0 564 859 A1, WO 2008/082170 A1, EP 1 849 493 A1, US 2004/039373 A1, US 2009/187140 A1, US 2006/129102 A1 und US 2005/090801 A1 genannt.

Der Erfindung liegt die Aufgabe zu Grunde, ein Set für die periphere Nervenblockade zur Verfügung zu stellen, welches eine einfache Handhabung und eine sichere Positionierung ermöglicht.

Die Aufgabe wird erfindungsgemäß gelöst durch ein Set gemäß dem Patentanspruch 1.

Vorteilhafte Ausführungen der Erfindung sind in den auf Patentanspruch 1 rückbezogenen Unteransprüchen angegeben.

Das erfindungsgemäße Set weist eine starre Kanüle auf, die für den Einstich in den peripheren Perineuralraum dient. Die Kanüle ist vorzugsweise in an sich bekannter Weise als unipolare Stimulationskanüle ausgebildet, die z.B. aus Stahl hergestellt ist und mit einer elektrisch isolierenden Beschichtung versehen ist, die lediglich die distale Spitze der Kanüle freilässt. Die exakte Position der distalen Kanülenspitze wird durch Elektrostimulation mittels eines proximal an die Kanüle angeschlossenen Stimulators bestimmt. Auf die Kanüle wird eine Katheterhülle aufgeschoben, die mittels der Kanüle in den Perineuralraum eingeführt wird. Die Katheterhülle ist als flexible nichtleitende schlauchförmige Hülle mit geringer Wandstärke, z.B. aus Teflon hergestellt. Damit das distale Ende der Katheterhülle in geeigneter Weise an der distalen Spitze der Kanüle anliegt, sitzt die Katheterhülle mit einer leichten radialen elastischen Vorspannung auf dem Außenumfang der Kanüle, wobei die distale Spitze der Kanüle für die elektrische Stimulation frei bleibt. Das distale Ende der Katheterhülle ist dünnwandig und distal konisch verjüngt, sodass die Katheterhülle zusammen mit der Kanüle das Körpergewebe leicht penetrieren kann.

Sobald die Kanüle mit der aufgeschobenen Katheterhülle im Perineuralraum positioniert ist, wird die Kanüle aus der Katheterhülle herausgezogen. Dann wird ein Innenkatheter durch die Katheterhülle eingeführt. Der Innenkatheter ist eine weichflexible Schlauchleitung, die zum Zuführen eines flüssigen Anästhetikums dient. Am proximalen Ende weist der Innenkatheter einen Spritzenanschluss zum Einleiten des Anästhetikums auf, während am distalen Ende wenigstens eine Austrittsöffnung vorgesehen ist.

Der Innenkatheter bewirkt eine Verstärkung der dünnwandigen flexiblen Katheterhülle, sodass die aus Katheterhülle und Innenkatheter bestehende Einheit knickfest ist. Eine präzise Positionierung der distalen Austrittsöffnung des Innenkatheters ist mittels der Katheterhülle möglich, indem der Innenkatheter in der Katheterhülle vorwärts zur distalen Spitze geschoben wird. Dabei kann der Innenkatheter auch so positioniert werden, dass er um eine variable Länge aus dem distalen Ende der Katheterhülle hinausragt. Das flexible weiche distale Ende des Innenkatheters, welches distal aus der Katheterhülle herausragt, ermöglicht ein Vorschieben in dem Perineuralraum, wobei die flexible weiche distale Spitze des Innenkatheters eventuellen Widerständen der Gewebestruktur ausweichen kann. Hierdurch wird das Risiko einer Schädigung des Nervengewebes beim Platzieren der Spitze wesentlich reduziert. Die variable axiale Anordnung des Innenkatheters in der Katheterhülle ermöglicht eine Anpassung der Länge der freien weichflexiblen Spitze des Innenkatheters an die anatomischen Bedingungen bei dem Vorschieben in den Perineuralraum. Das Set verbindet daher eine optimale Glätte und Dünne der Katheterhülle über deren gesamte Länge für das Vorschieben durch die Haut und das Gewebe mit der weichen Flexibilität der distalen Spitze des Innenkatheters für eine Vermeidung von Schädigungen der Gewebestruktur.

Der Innenkatheter weist ein proximales Konnektorteil auf, welches direkt mit dem Konnektorteil am proximalen Ende der Katheterhülle konnektiert werden kann. Proximal von diesem Konnektorteil setzt sich der Innenkatheter in einem Zuspritzschlauch fort, der an seinem proximalen Ende einen Spritzenanschluss aufweist.

Die direkte Konnektierung des Innenkatheters mit der Katheterhülle bedeutet eine Zeitersparnis und Erleichterung bei der Handhabung, da die Anzahl der Schritte für das Zusammensetzen der Katheterhüllen-Innenkather-Einheit reduziert wird. Außerdem kann dadurch ein versehentliches falsches Anschließen ausgeschlossen werden. Da der Innenkatheter an dem Konnektorteil unmittelbar in den Zuspritzschlauch übergeht, kann über den Spritzenanschluss direkt zugespritzt werden, ohne dass ein zusätzlicher Katheteradapter notwendig ist. Bei dem Konnektieren des Innenkatheter mit der Katheterhülle wird der Zuspritzschlauch mit dem Spritzenanschluss ohne zusätzliche Bauteile und ohne zusätzliche Handgriffe des Benutzers insbesondere für eine kontinuierliche Nervenblockade konnektiert.

Beim Platzieren des Innenkatheters kann über den Zuspritzschlauch eine Flüssigkeit zugespritzt werden. Der Benutzer kann die Ausbreitung der Flüssigkeit in dem Perineuralraum mittels Ultraschall beobachten. Die Flüssigkeit dient zum Dilatieren des Nervenkanals oder Perineuralraums, um eine reibungsfreie Vorwärtsbewegung der distalen Spitze des Innenkatheters zu ermöglichen. Die Flüssigkeit kann beispielsweise ein Anästhetikum oder eine Lösung von z.B. Dextrose in Wasser sein.

In einer vorteilhaften Ausführung weist der Innenkatheter nicht nur wenigstens eine distale Austrittsöffnung auf, sondern zusätzlich auch noch wenigstens eine proximale Austrittsöffnung in seiner Wandung. Diese wenigstens eine proximale Austrittsöffnung ist axial in der Wandung des Innenkatheters so angeordnet, dass sie beim Einführen des Innenkatheters in die Katheterhülle in den Ringraum zwischen der Außenoberfläche des Innenkatheters und der Innenoberfläche der Katheterhülle mündet. Wird über den Innenkatheter eine Flüssigkeit eingeleitet, so tritt diese Flüssigkeit nicht nur durch die distalen Austrittsöffnungen, sondern auch durch die proximale Austrittsöffnung aus. Die Flüssigkeit füllt somit den Ringraum zwischen Innenkatheter und Katheterhülle über die gesamte axiale Länge zwischen der proximalen Austrittsöffnung des Innenkatheters und der distalen Öffnung der Katheterhülle. Die diesen Ringraum ausfüllende Flüssigkeit bewirkt eine deutliche Ultraschallsichtbarkeit. Die aus Katheterhülle und Innenkatheter bestehende Einheit wird dadurch über ihre gesamte Länge deutlich ultraschall-sichtbar, was eine Positionierung und eine eventuelle Lagekorrektur erleichtert. Durch abwechselndes Injizieren und Aspirieren der Flüssigkeit kann die Ultraschall-Reflexion des Ringraums variiert werden, wodurch die Ultraschall-Erkennbarkeit zusätzlich verbessert wird.

Im Folgenden wird die Erfindung anhand von in der Zeichnung dargestellten Ausführungsbeispielen näher erläutert. Es zeigen:
- Fig. 1: eine Kanüle des Sets,
- Fig. 2: eine Katheterhülle des Sets,
- Fig. 3: einen Innenkatheter des Sets,
- Fig. 4: die Kanüle mit aufgeschobener Katheterhülle,
- Fig. 5: die Katheterhülle mit eingeführtem Innenkatheter,
- Fig. 6: den Innenkatheter in einer zweiten Ausführung und
- Fig. 7: die Katheterhülle mit eingeführtem Innenkatheter in der zweiten Ausführung.

Das Set für die periphere Nervenblockade besteht aus einer Kanüle 10, einer Katheterhülle 20 und einem Innenkatheter 30.

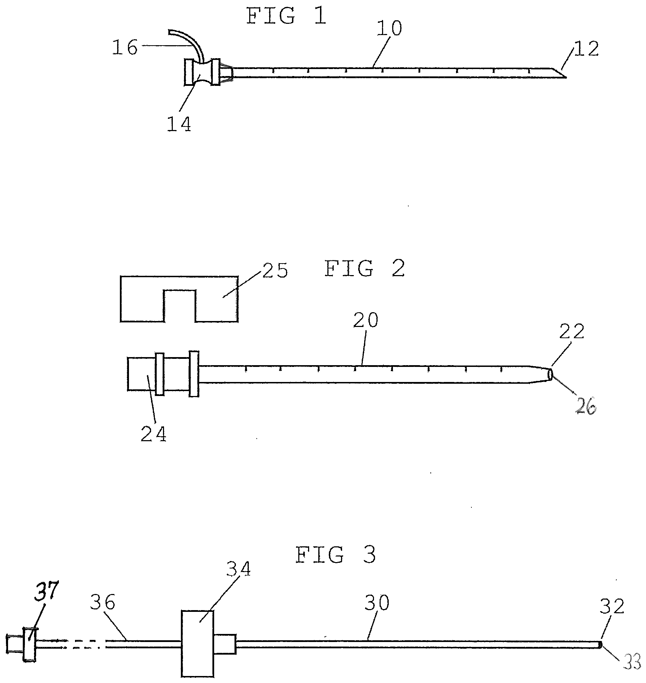

Die beispielsweise in Figur 1 dargestellte Kanüle 10 ist vorzugsweise als unipolare Stimulationskanüle ausgebildet. Sie weist einen starren, vorzugsweise aus Stahl gefertigten rohrförmigen Schaft auf, dessen distale Spitze 12 in an sich bekannter Weise mit einem Facettenschliff als Tuohy-Spitze oder Sprottespitze ausgebildet sein kann. Die Kanüle 10 ist über ihre gesamte Länge mit einer äußeren elektrisch isolierenden Beschichtung versehen, die lediglich die distale Spitze 12 freilässt. Am proximalen Ende der Kanüle 10 ist ein Ansatz 14 aus Kunststoff angebracht, durch welchen ein Anschlusskabel 16 hindurchgeführt ist. Das Anschlusskabel 16 kontaktiert die metallische elektrisch leitende Kanüle 10 und dient zum Anschluss eines elektrischen Stimulators.

Die beispielsweise in Figur 2 dargestellte Katheterhülle 20 ist ein flexibler Katheter aus einem nichtleitenden weichen Kunststoff. Das distale Ende 22 der Katheterhülle 20 ist sich distalwärts konisch verjüngend ausgebildet. Am distalen Ende 22 weist die Katheterhülle 20 eine koaxiale Austrittsöffnung 26 auf. Am proximalen Ende der Katheterhülle 20 ist ein erstes Konnektorteil 24 angeordnet, welches als weibliches Luer-Lock-Konnektorteil ausgebildet ist. Auf das Konnektorteil 24 kann ein Flügel 25 abnehmbar oder nicht abnehmbar aufgesetzt sein.

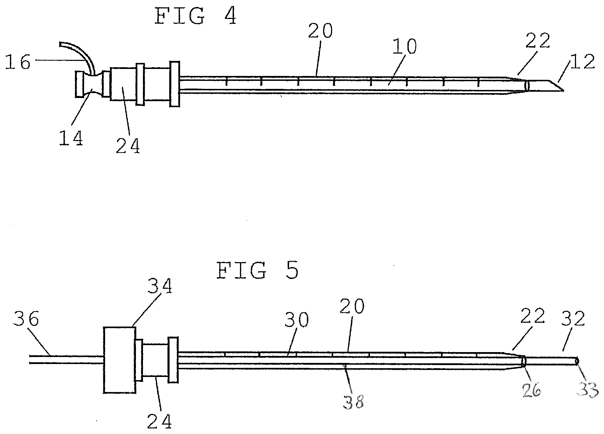

Wie Figur 4 zeigt, kann die Katheterhülle 20 vorzugsweise mit Hilfe des Flügels 25 auf die Kanüle 10 aufgeschoben werden. Die Länge der Kanüle 10 und die Länge der Katheterhülle 20 sind so aufeinander abgestimmt, dass die distale Spitze 12 der Kanüle 10 distal aus dem distalen Ende 22 der Katheterhülle 20 hinausragt, wenn die Katheterhülle 20 vollständig auf die Kanüle 10 aufgeschoben ist und der proximale Ansatz 14 der Kanüle 10 in das Konnektorteil 24 der Katheterhülle 20 eingreift. Der Durchmesser des distalen Endes 22 der Katheterhülle 20 ist so dimensioniert, dass das distale Ende 22 unter einer leichten elastischen Vorspannung radial am Außenumfang der Kanüle 10 anliegt. Das dichte Anliegen des distalen Endes 22 und dessen konische Ausbildung bewirken einen stufenlosen Übergang von der distalen Spitze 12 der Kanüle 10 zum distalen Ende 22 der Katheterhülle 20. Dadurch wird ein leichtes Eindringen der Kanüle 10 mit der aufgeschobenen Katheterhülle 20 durch die Haut und das Gewebe des Patienten beim Einstich ermöglicht.

Der beispielsweise in Figur 3 dargestellte Innenkatheter 30 besteht aus einer weich flexiblen Schlauchleitung, deren Außendurchmesser geringfügig kleiner ist als der Innendurchmesser der Katheterhülle 20. Am distalen Ende 32 weist der Innenkatheter 30 wenigstens eine Austrittsöffnung 33 auf. Im dargestellten Ausführungsbeispiel ist eine einzige koaxiale Austrittsöffnung 33 vorgesehen. Im Rahmen der Erfindung können auch radiale Austrittsöffnungen zusätzlich oder ausschließlich vorgesehen sein. Am proximalen Ende des Innenkatheters 30 ist ein zweites Konnektorteil 34 angeordnet. Das zweite Konnektorteil 34 des Innenkatheters 30 ist komplementär zu dem ersten Konnektoreil 24 der Katheterhülle 20 ausgebildet, d. h. im dargestellten Ausführungsbeispiel als männliches Luer-Lock-Konnektorteil. Der Innenkatheter 30 ist als hohler Schlauch aus Kunststoff ausgebildet und kann gegebenenfalls auch einen metallischen Versteifungsdraht, einen Spiraldraht und/oder einen Stimulationsdraht, enthalten. Der Innenkatheter 30 ist elastisch biegsam aber ausreichend stabil gegen ein Verbiegen, Ausbeulen oder Knicken. In dem Konnektorteil 34 geht der Innenkatheter 30 in einen proximal anschließenden Zuspritzschlauch 36 über, der an seinem proximalen Ende einen Spritzenanschluss 37 aufweist. Über den Spritzenanschluss 37 kann mittels einer nicht dargestellten Spritze eine Flüssigkeit in den Innenkatheter 30 zugeführt werden.

Wie Figur 5 zeigt, sind die Länge der Katheterhülle 20 und die Länge des Innenkatheters 30 so aufeinander abgestimmt, dass das distale Ende 32 des Innenkatheters 30 distal aus dem distalen Ende 22 der Katheterhülle 20 herausragt, wenn der Innenkatheter 20 vollständig soweit in die Katheterhülle 20 eingeführt ist, dass das zweite Konnektorteil 34 des Innenkatheters 30 mit dem ersten Konnektorteil 24 der Katheterhülle 20 in Eingriff kommt und die zwei Konnektorteile 24 und 34 miteinander verbunden werden. Da der Innendurchmesser der Katheterhülle 20 geringfügig größer ist als der Außendurchmesser des Innenkatheters 30 verbleibt zwischen dem Außerdurchmesser des Innenkatheters 30 und dem Innendurchmesser der Katheterhülle 20 ein Ringraum 38.

Ein beispielhaftes Verfahren für die periphere Nervenblockade mit dem dargestellten Set verläuft folgendermaßen:
Zunächst wird die Katheterhülle 20 auf die Kanüle 10 aufgeschoben, bis das Konnektorteil 24 mit dem Ansatz 14 der Kanüle 10 in Eingriff kommt. Die distale Spitze 12 der Kanüle 10 ragt dabei aus dem distalen Ende 22 der Katheterhülle 20 heraus, wie in Figur 4 gezeigt ist. Die Kanüle 10 wird mittels des Anschlusskabels 16 an einen elektrischen Nervenstimulator angeschlossen. Nun wird die Kanüle 10 durch die Haut und das Gewebe des Patienten in den Perineuralraum eingestochen, wobei die Positionierung der Spitze 12 der Kanüle mittels der elektrischen Nervenstimulation kontrolliert wird. Da das distale Ende 22 der Katheterhülle 20 elastisch vorgespannt an der Kanüle 10 anliegt und konisch ausgebildet ist, kann die Katheterhülle 20 ohne zusätzlichen Widerstand zusammen mit der Kanüle 10 eingestochen werden.

Sobald die Spitze 12 der Kanüle 10 im Perineuralraum positioniert ist, kann die Katheterhülle 20 bei Bedarf auf der Kanüle 10 in distaler Richtung vorgeschoben werden, sodass das distale Ende 22 der Katheterhülle 20 über die Spitze 12 der Kanüle 10 hinaus weiter in den Perineuralraum vorgeschoben wird. Sobald die Katheterhülle 20 mit einem solchen distalen Vorschieben oder auch ohne ein solches distales Vorschieben positioniert ist, wird die Kanüle 10 aus der Katheterhülle 20 herausgezogen. Nachdem die Kanüle 10 aus der Katheterhülle 20 herausgezogen ist, wird der Innenkatheter 30 vom proximalen Ende her in die Katheterhülle 20 eingeführt. Zunächst wird der Innenkatheter 30 soweit in die Katheterhülle 20 eingeführt, bis sein distales Ende 32 aus dem distalen Ende 22 der Katheterhülle 20 austritt. Bei diesem Vorschieben des Innenkatheters 30 kann über den Zuspritzschlauch 36 eine Flüssigkeit in den Innenkatheter 30 zugeführt werden, die an dessen distaler Austrittsöffnung 33 austritt. Diese Flüssigkeit kann dazu dienen, den Raum aufzuweiten, durch welchen der Innenkatheter 30 gegebenenfalls zusammen mit der Katheterhülle 20 vorgeschoben wird. Die Flüssigkeit bildet somit ein Polster, welches die Vorwärtsbewegung des distalen Endes 32 erleichtert. Als Flüssigkeit kann beispielsweise eine wässrige Dextroselösung dienen.

Bei dem gemeinsamen Voschieben von Katheterhülle 20 und Innenkatheter 30 dient der Innenkatheter 30 dazu, die weich flexible Katheterhülle 20 über deren gesamte Länge zu verstärken, sodass ein Knicken und Ausbiegen der aus Katheterhülle 20 und Innenkatheter 30 bestehenden Kathetereinheit vermieden wird. Die distal aus der Katheterhülle 20 herausragende Länge des distalen Endes 32 kann dabei variiert werden, so dass die Kathetereinheit eine durch das überstehende distale Ende 32 des Innenkatheters 30 gebildete distale Spitze aufweist, die eine weichere Flexibilität aufweist und Gewebsstrukturen ausweichen kann, ohne diese zu schädigen. Die Länge des überstehenden distalen Endes 32 kann dabei den jeweiligen anatomischen Gegebenheiten des Einsatzes angepasst werden.

Die aus der distalen Austrittsöffnung 33 des Innenkatheters 30 austretende Flüssigkeit erlaubt eine Beobachtung der Position des distalen Endes 32 des Innenkatheters und damit der aus Innenkatheter 30 und Katheterhülle 20 bestehenden Kathetereinheit mittels Ultraschall.

Sobald das distale Ende 32 des Innenkatheters 30 optimal im Perineuralraum positioniert ist, wird die Katheterhülle 20 proximal zurückgezogen, bis das erste Konnektorteil 24 der Katheterhülle 20 mit dem zweiten Konnektorteil 34 des Innenkatheters 30 in Eingriff kommt, worauf die beiden Konnektorteile 24 und 34 konnektiert werden. Die aus der Konnektorhülle 20 und dem mit dieser konnektierten Innenkatheter 30 bestehende Kathetereinheit kann nun in ihrer Position verbleiben für eine kontinuierliche Anästhesie oder auch für eine eventuelle spätere Nachdosierung. Die Zuführung eines Anästhetikums erfolgt dabei über den Spritzenanschluss 37 und den Zuspritzschlauch 36.

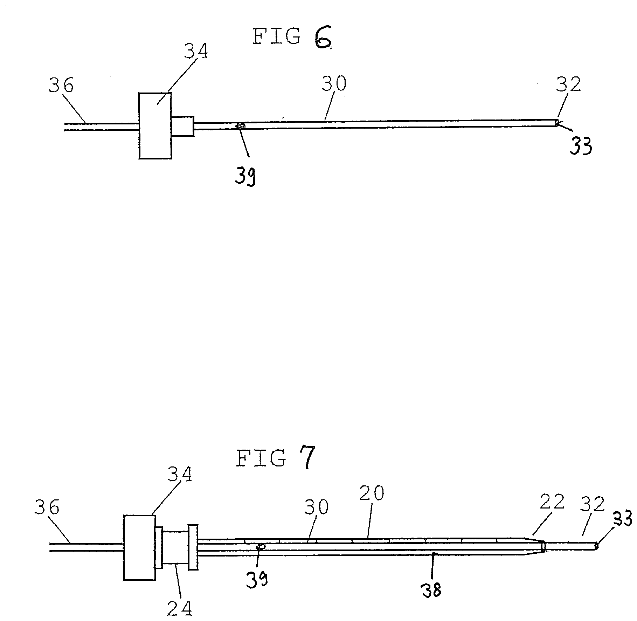

In den Figuren 6 und 7 ist eine abgewandelte zweite Ausführung des Innenkatheters 30 dargestellt. In dieser Ausführung stimmt der Innenkatheter 30 mit der vorangehend beschriebenen Ausführung der Figur 3 überein und unterscheidet sich nur darin, dass zusätzlich zu der wenigstens einen Austrittsöffnung 33 am distalen Ende 32 noch wenigstens eine proximale Austrittsöffnung 39 in der Wandung des Innenkatheters 30 vorgesehen ist. Die proximale Austrittsöffnung 39 ist dabei in einer solchen axialen Position in der Nähe des zweiten Konnektorteils 34 angeordnet, dass diese proximale Austrittsöffnung 39 in den Ringraum 38 zwischen Innenkatheter 30 und Katheterhülle 20 mündet, wenn der Innenkatheter 30 in die Katheterhülle 20 eingeführt ist. Anstelle einer proximalen Austrittsöffnung 39 können auch mehrere proximale Austrittsöffnungen vorgesehen sein.

Ist der Innenkatheter 30 in die Katheterhülle 20 eingeführt, wie dies in Figur 7 gezeigt ist, so kann über den Zuspritzschlauch 36 eine Flüssigkeit zugeführt werden. Diese Flüssigkeit tritt über die distale Austrittsöffnung 33 aus, wie dies in dem ersten Ausführungsbeispiel beschrieben ist. Außerdem tritt die Flüssigkeit über die proximale Austrittsöffnung 39 aus und fließt in dem Ringraum 38 in distaler Richtung, wo sie über die Austrittsöffnung 26 am distalen Ende 22 der Katheterhülle austritt. Die an der distalen Austrittsöffnung 33 austretende Flüssigkeit erlaubt eine Ultraschall-Beobachtung des distalen Endes 32 des Innenkatheters 30. Die Flüssigkeit in dem Ringraum 38 ermöglicht eine verbesserte UltraschallSichtbarkeit über die gesamte Länge des Ringraums 38, d. h. über die gesamte Länge der Katheterhülle 20. Dadurch ergibt sich der Vorteil, dass nicht nur die Position des distalen Endes 32 des Innenkatheters 30 verbessert mittels Ultraschall beobachtbar ist, sondern dass zusätzlich auch die Lage der aus der Katheterhülle 20 und Innenkatheter 30 bestehenden Kathetereinheit über deren gesamte Länge verbessert ultraschall-sichtbar ist. Die Ultraschall-Beobachtung der Kathetereinheit über deren gesamte Länge kann zusätzlich noch dadurch verbessert werden, dass die Flüssigkeit über den Zuspritzschlauch 36 abwechselnd injiziert und aspiriert wird. Dadurch wird eine pulsierende axiale Bewegung der Flüssigkeit in dem Ringraum 38 erzeugt, die eine sich ändernde Ultraschall-Reflexion bewirkt und dadurch die Kathetereinheit deutlicher erkennbar macht.

### Bezugszeichenliste

- 10: Kanüle
- 12: Spritze
- 14: Ansatz
- 16: Anschlusskabel

- 20: Katheterhülle
- 22: distales Ende
- 24: erstes Konnektorteil
- 25: Flügel
- 26: Austrittsöffnung

- 30: Innenkatheter
- 32: distales Ende
- 33: Austrittsöffnung
- 34: zweites Konnektorteil
- 36: Zuspritzschlauch
- 37: Spritzenanschluss
- 38: Ringraum
- 39: Austrittsöffnung

## Patentansprüche

1. Set für die peripheren Nervenblockade, mit einer starren Kanüle (10) für den Einstich in den Perineuralraum, mit einer äußeren Katheterhülle (20), die auf die Kanüle (10) aufschiebbar ist, wobei die distale Spitze (12) der Kanüle (10) über das distale Ende (22) der Katheterhülle (20) hinausragt, mit einem Innenkatheter (30), der nach dem Herausziehen der Kanüle (10) aus der Katheterhülle (20) durch diese Katheterhülle (20) einführbar ist und am distalen Ende (32) wenigstens eine Austrittsöffnung (33) aufweist, und mit einem ersten Konnektorteil (24) am proximalen Ende der Katheterhülle,
**dadurch gekennzeichnet, dass** der Innenkatheter (30) ein zu dem ersten Konnektorteil (24) komplementäres zweites Konnektorteil (34) aufweist und dass der Innenkatheter (30) durch Konnektieren des ersten Konnektorteils (24) und des zweiten Konnektorteils (34) in der Katheterhülle (20) in einer axialen Position festlegbar ist, in welcher das distale Ende (32) des Innenkatheters (30) mit einer die wenigstens eine Austrittsöffnung (33) aufweisenden vorgegebenen Länge aus dem distalen Ende (22) der Katheterhülle hinausragt.

2. Set nach Anspruch 1,
**dadurch gekennzeichnet, dass** in dem zweiten Konnektorteil (34) ein Zuspritzschlauch (36) proximal an den Innenkatheter (30) anschließt.

3. Set nach Anspruch 1,
**dadurch gekennzeichnet, dass** die Katheterhülle (20) aus einem weichen flexiblen Kunststoff besteht.

4. Set nach Anspruch 3,
**dadurch gekennzeichnet, dass** die Katheterhülle (20) am distalen Ende (22) sich distalwärts konisch verjüngt.

5. Set nach Anspruch 3 oder 4,
**dadurch gekennzeichnet, dass** die Katheterhülle (20) zumindest mit ihrem distalen Ende (22) elastisch vorgespannt am Außenumfang der Kanüle (10) anliegt.

6. Set nach Anspruch 1,
**dadurch gekennzeichnet, dass** das erste Konnektorteil (24) der Katheterhülle (20) ein weibliches Luer-Lock-Konnektorteil und das zweite Konnektorteil (34) des Innenkatheters (30) ein männliches Luer-Lock-Konnektorteil ist.

7. Set nach Anspruch 1,
**dadurch gekennzeichnet, dass** die Katheterhülle (20) an ihrem proximalen Ende einen Flügel (25) aufweist.

8. Set nach einem der vorhergehenden Ansprüche,
**dadurch gekennzeichnet, dass** der Innenkatheter (30) aus einem flexiblen Kunststoff besteht.

9. Set nach den Ansprüchen 3 und 8,
**dadurch gekennzeichnet, dass** der eingeführte Innenkatheter (30) die Katheterhülle (20) verstärkt, insbesondere gegen ein Knicken versteift.

10. Set nach einem der vorhergehenden Ansprüche,
**dadurch gekennzeichnet, dass** der Innenkatheter (30) zusätzlich zu der wenigstens einen distalen Austrittsöffnung (33) wenigstens eine proximale Austrittsöffnung (39) in seiner Wandung aufweist, die bei in die Katheterhülle (20) eingeführtem Innenkatheter (30) in den Ringraum (38) zwischen der Außenoberfläche des Innenkatheters (30) und der Innenoberfläche der Katheterhülle (20) mündet.

## Claims

1. Set for peripheral nerve blocks with a rigid cannula (10) for insertion into the perineural space, with an outer catheter sheath (20) which can be slid onto the cannula (10), wherein the distal tip (12) of the cannula (10) extends beyond the distal end (22) of the catheter sheath (20), with an inner catheter (30) that can be inserted through the catheter sheath (20) after removing the cannula (10) from the catheter sheath (20), and which comprises at least one outlet opening (33) at its distal end (32), and with a first connecting part (24) at the proximal end of the catheter sheath (20), **characterized in that** the inner catheter (30) comprises a second connecting part (34) that complements the first connecting part (24) and **in that** the inner catheter (30) can be fixed, by means of contacting the first connecting part (24) and the second connecting part (34), in the catheter sheath (20) in an axial position in which the distal end (32) of the inner catheter (30) protrudes from the distal end (22) of the catheter sheath (20) by a specified length that comprises at least one outlet opening (33).

2. Set in accordance with claim 1,
**characterized in that** an injecting tube (36) proximally joins with the inner catheter (30) in the second connecting part (34).

3. Set in accordance with claim 1,
**characterized in that** the catheter sheath (20) consists of a soft, flexible plastic material.

4. Set in accordance with claim 3,
**characterized in that** the catheter sheath (20) tapers conically at the distal end (22) in the distal direction.

5. Set in accordance with claim 3 or 4,
**characterized in that** the catheter sheath (20) joins, resiliently biased, with the outer periphery of the cannula (10) at least at its distal end (22) .

6. Set in accordance with claim 1,
**characterized in that** the first connecting part (24) of the catheter sheath (20) is a female Luer lock connecting part and the second connecting part (34) of the inner catheter (30) is a male Luer lock connecting part.

7. Set in accordance with claim 1,
**characterized in that** the catheter sheath (20) comprises a wing (25) at its proximal end.

8. Set in accordance with any of the preceding claims,
**characterized in that** the inner catheter (30) consists of a flexible plastic material.

9. Set in accordance with the claim 3 and 8,
**characterized in that** the inserted inner catheter (30) strengthens the catheter sheath (20), in particular stiffening it against buckling.

10. Set in accordance with any of the preceding claims,
**characterized in that** the inner catheter (30) comprises, in addition to the at least one distal outlet opening (33), at least one proximal outlet opening (39) in its inner wall which opens into the ring room (38) between the outer surface of the inner catheter (30) and the inner surface of the catheter sheath (20) when the inner catheter (30) is inserted in the catheter sheath (20).

## Revendications

1. Kit pour le blocage nerveux périphérique comprenant une canule rigide (10) pour une incision dans l'espace périneural avec un manchon extérieur de cathéter (20) qui est emmanché sur la canule (10),
la pointe distale (12) de la canule (10) dépassant de l'extrémité distale (22) du manchon de cathéter (20),
un cathéter interne (30) qui, après extraction de la canule (10) hors du manchon de cathéter (20), s'introduit par ce manchon de cathéter (20) et comporte à l'extrémité distale (32) au moins un orifice de sortie (33) ainsi qu'une première pièce de connexion (24) à l'extrémité proximale du manchon de cathéter,
kit **caractérisé en ce que**
le cathéter interne (30) a une seconde partie de connecteur (34) complémentaire à la première partie de connecteur (24), et
le cathéter interne (30) se fixe dans une position axiale par la connexion de la première partie de connecteur (24) et de la seconde partie de connecteur (34) dans le manchon de cathéter (20),
position dans laquelle l'extrémité distale (32) du cathéter interne (30) dépasse de l'extrémité distale (22) du manchon de cathéter d'au moins une longueur prédéfinie par rapport à l'orifice de sortie (33).

2. Kit selon la revendication 1,
**caractérisé en ce que**
un tube d'injection (36) est relié à l'extrémité proximale du cathéter interne (30) dans la seconde partie de connecteur (34).

3. Kit selon la revendication 1,
**caractérisé en ce que**
le manchon de cathéter (20) est en une matière synthétique flexible, molle.

4. Kit selon la revendication 3,
**caractérisé en ce que**
l'extrémité distale (22) du manchon de cathéter (20) diminue de façon conique dans la direction distale.

5. Kit selon la revendication 3 ou 4,
**caractérisé en ce que**
le manchon de cathéter (20) s'applique de façon précontrainte élastiquement contre la périphérie extérieure de la canule (10) par son extrémité distale (22).

6. Kit selon la revendication 1,
**caractérisé en ce que**
la première partie de connecteur (24) du manchon de cathéter (20) comporte une pièce de connecteur Luer femelle et la seconde partie de connecteur (34) du cathéter interne (30) comporte une pièce Luer mâle de connecteur.

7. Kit selon la revendication 1,
**caractérisé en ce que**
l'extrémité proximale du manchon de cathéter (20) comporte une ailette (25).

8. Kit selon l'une des revendications précédentes,
**caractérisé en ce que**
le cathéter interne (30) est en une matière synthétique flexible.

9. Kit selon les revendications 3 et 8,
**caractérisé en ce que**
le cathéter interne (30) engagé, renforce le manchon de cathéter (20), notamment contre le pliage.

10. Kit selon l'une des revendications précédentes,
**caractérisé en ce que**
le cathéter interne (30) comporte en plus d'au moins un orifice de sortie distale (33) au moins un orifice de sortie proximale (39) dans sa paroi, et qui, lorsque le cathéter interne (30) est engagé dans le manchon de cathéter (20), débouche dans le volume annulaire (38) entre la surface extérieure du cathéter interne (30) et la surface intérieure du manchon de cathéter (20).
